# FASCICULE DE BREVET EUROPEEN

(11) **EP 3 316 845 B1**
(45) Date de publication et mention de la délivrance du brevet: **07.10.2020**
(21) Numéro de dépôt: 16750899.3
(22) Date de dépôt: 28.06.2016
(51) Int. Cl.: A61H 39/08

(54) **DISPOSITIF D'IMPLANTATION COMPRENANT UN ENSEMBLE D'IMPLANTS**
IMPLANTATIONSVORRICHTUNG MIT EINEM SATZ AUS IMPLANTATEN
IMPLANTATION DEVICE COMPRISING A SET OF IMPLANTS

(30) Priorité: 02.07.2015 FR 1556235
(43) Date de publication de la demande: 09.05.2018
(73) Titulaire: Sedatelec, 69540 Irigny (FR)
(72) Inventeur: DUMONT, François, 42140 Maringes (FR)
(74) Mandataire: Chevalier, Renaud Philippe
(86) Numéro de dépôt international: PCT/FR2016/051608
(87) Numéro de publication internationale: WO 2017/001773

(56) Documents cités:
- WO-A1-01/39829
- CN-U- 2 083 927
- CN-U- 201 880 014
- CN-U- 203 790 289
- CN-U- 204 293 501
- ES-U- 272 596
- KR-A- 20140 010 513
- KR-A- 20140 143 601
- US-A1- 2005 067 309

## Description

La présente invention concerne un dispositif d'implantation pour implanter des implants dans le corps d'un être vivant, humain ou animal. La présente invention concerne en particulier un dispositif d'implantation pour implanter des aiguilles d'acupuncture, par exemple des aiguilles d'acupuncture du type semi-permanente ou du type permanente.

La présente invention s'applique au domaine de l'implantation d'implants, par exemple d'implants médicaux, en particulier au domaine de l'implantation d'aiguilles d'acupuncture, et plus particulièrement au domaine de l'implantation d'aiguilles d'acupuncture semi-permanentes. Par exemple, la présente invention s'applique à l'implantation d'aiguilles configurées pour l'implantation dans le pavillon auriculaire. Le document ES272596U décrit un injecteur multiple pour aiguilles d'auriculo-médecine et acupuncture avec toutes les caractéristiques techniques du préambule de la revendication 1.

Une aiguille d'acupuncture reste implantée seulement pendant la séance d'acupuncture, puis l'aiguille d'acupuncture est retirée à la fin de la séance d'acupuncture. Au contraire, une aiguille semi-permanente peut rester implantée après la séance d'acupuncture, puis être retirée ultérieurement.

Des aiguilles semi-permanentes ou des aiguilles permanentes peuvent être implantées dans les corps d'êtres vivants humains ou animaux.

FR2335201A1 illustre un dispositif d'implantation pour implanter une aiguille d'acupuncture semi-permanente. Le dispositif d'implantation comprend un poussoir pour permettre au praticien de générer un effort de poussée, un membre de transmission pour transmettre l'effort de poussée à l'aiguille d'acupuncture semi-permanente, ainsi qu'un guide pour guider l'aiguille d'acupuncture jusqu'à un orifice de sortie placé contre l'épiderme d'un patient.

Le dispositif d'implantation et l'aiguille d'acupuncture sont fournis à l'état stérile et ils sont prévus pour un usage unique. Après avoir implanté l'aiguille d'acupuncture, le praticien recycle ou jette le dispositif d'implantation.

Cependant, pour implanter plusieurs aiguilles d'acupuncture, le praticien doit déballer plusieurs aiguilles puis les implanter, ce qui induit une perte de temps. En outre, ces multiples déballages d'aiguilles induisent une rupture du rythme du geste du praticien, ce qui risque de générer une imprécision lors des implantations successives.

La présente invention a notamment pour but de résoudre, en tout ou partie, les problèmes mentionnés ci-avant.

Dans ce but, la présente invention a pour objet un dispositif d'implantation, pour implanter des implants, par exemple des aiguilles d'acupuncture du type semi-permanente, dans le corps d'un être vivant, le dispositif d'implantation comprenant au moins :
- un ensemble d'implants comprenant au moins deux implants disposés successivement selon une ligne d'implants, les implants étant reliés de sorte que chaque implant peut être séparé de l'ensemble d'implants,
- un organe de guidage définissant un passage qui s'étend selon la ligne d'implants et jusqu'à un orifice de sortie destiné à être placé contre l'épiderme de l'être vivant, le passage étant configuré pour guider l'ensemble d'implants en translation vers l'orifice de sortie et selon la ligne d'implants,
- un membre de transmission agencé pour transmettre un effort de poussée à l'ensemble d'implants de façon à déplacer l'ensemble d'implants en translation vers l'orifice de sortie et selon la ligne d'implants, le membre de transmission comportant plusieurs saillies disposées successivement selon la ligne d'implants,
- un organe de poussée configuré pour permettre à un praticien de générer l'effort de poussée, l'organe de poussée étant déplaçable entre une position de repos et une position de poussée, l'organe de poussée comportant une partie d'appui configurée pour appuyer successivement contre plusieurs des saillies lors de plusieurs déplacements successifs de l'organe de poussée entre la position de repos et la position de poussée, la partie d'appui étant configurée pour présenter successivement :
   - une position d'appui, dans laquelle la partie d'appui appuie contre une saillie respective lors de chaque déplacement de l'organe de poussée entre la position de repos et la position de poussée, et
   - une position de franchissement, dans laquelle la partie d'appui est déformée élastiquement par la saillie respective de sorte que la partie d'appui peut franchir la saillie lorsque l'organe de poussée subit un effort de rappel vers sa position de repos,
le dispositif d'implantation comprenant en outre un élément d'arrêt unidirectionnel qui est relié à l'organe de guidage et qui est configuré pour présenter successivement :
- une configuration de franchissement, dans laquelle l'élément d'arrêt unidirectionnel est déformé élastiquement par une des saillies de sorte que la saillie peut franchir l'élément d'arrêt unidirectionnel, et
- une configuration d'arrêt, dans laquelle l'élément d'arrêt unidirectionnel bute contre une des saillies de façon à immobiliser le membre de transmission en sens inverse de la translation vers l'orifice de sortie et selon la ligne d'implants.

Ainsi, un tel dispositif d'implantation permet au praticien d'implanter plusieurs implants rapidement et simplement, car le dispositif d'implantation est facilement manipulable. Donc un tel dispositif d'implantation diminue la durée de pose des implants, ce qui augmente le confort de l'être vivant, par exemple du patient.

De plus, un tel dispositif d'implantation réduit le coût d'implantation de plusieurs implants, puisqu'un seul dispositif d'implantation stérile permet d'implanter plusieurs implants, tandis qu'un dispositif d'implantation stérile de l'art antérieur ne permettait d'implanter qu'un seul implant.

Par ailleurs, pour poser chaque implant de l'ensemble d'implants, le praticien exerce le même effort de poussée, ce qui augmente la précision de la pose des implants, car chaque dispositif d'implantation unique de l'art antérieur nécessite un effort de poussée plus ou moins spécifique, en fonction de la reproductibilité dans la fabrication des dispositifs d'implantation unique. Le praticien peut exercer l'effort de poussée à la main.

Dans la présente demande, l'expression « selon une direction de référence » se rapporte à une direction parallèle ou colinéaire à une direction de référence. De même, l'expression « selon une ligne de référence » se rapporte à une ligne parallèle ou colinéaire à une ligne de référence. Par exemple, l'expression « selon la ligne d'implants » se rapporte à une ligne, curviligne ou rectiligne, parallèle ou colinéaire à la ligne d'implants.

Selon une variante, la ligne d'implants est droite. Dans cette variante, la translation de l'ensemble d'implants est rectiligne. Dans cette variante, les saillies successives peuvent être disposées parallèlement à la ligne d'implants. En d'autres termes, le membre de transmission forme une sorte de tige dentelée, dont les dents sont formées par les saillies.

Alternativement à cette variante, la ligne d'implants est courbe avec une ou plusieurs courbures. Dans cette variante, la translation de l'ensemble d'implants est curviligne. Dans cette variante, les dimensions et les matériaux des composants du dispositif d'implantation sont sélectionnés de sorte que l'ensemble d'implants peut glisser dans le passage. Le ou chaque rayon de courbure peut par exemple être supérieur à 80 mm, de façon à permettre une translation de l'ensemble d'implants avec peu de frottements contre le passage.

Dans cette variante, le membre de poussée est configuré pour se déformer pendant la translation curviligne. Pour obtenir cette déformation de l'organe de poussée, on peut par exemple former l'organe de poussée en sélectionnant une section transversale ayant un diamètre de 1,3 mm, une longueur de 20 mm et un matériau thermoplastique tel qu'un Acrylonitrile Butadiène Styrène (ABS) ou un Polycarbonate (PC) qui a ainsi un module d'Young approprié. En d'autres termes, la ligne d'implants peut être faiblement incurvée. Dans cette variante, les saillies peuvent être disposées sur la ligne d'implants courbe.

Selon une variante, le passage est directement en contact avec l'ensemble d'implants. Dans cette variante, il n'y a aucun composant intermédiaire entre l'ensemble d'implants et le passage. Ainsi, le dispositif d'implantation peut être particulièrement compact selon des directions perpendiculaires à la ligne d'implants.

Selon une variante, l'organe de poussée comprend une partie de préhension configurée pour permettre au praticien de manipuler le dispositif d'implantation.

Selon une variante, l'élément d'arrêt unidirectionnel est solidaire de l'organe de guidage. Par exemple, l'élément d'arrêt unidirectionnel et l'organe de guidage peuvent être monoblocs. Alternativement à cet exemple, l'élément d'arrêt unidirectionnel et l'organe de guidage peuvent être liés par encliquetage élastique ou par emmanchement.

Selon une variante, l'organe de guidage est composé d'une seule pièce. Alternativement à ce mode de réalisation, l'organe de guidage peut être composé d'au moins deux pièces assemblées.

Selon une variante, l'organe de poussée est composé d'une seule pièce. Alternativement à ce mode de réalisation, l'organe de poussée peut être composé d'au moins deux pièces assemblées.

Selon un mode de réalisation, les implants sont en contact deux à deux dans l'ensemble d'implants.

En d'autres termes, les implants sont contigus deux à deux dans l'ensemble d'implants. Ainsi, l'ensemble d'implants est simple à assembler et il est particulièrement compact.

Alternativement à ce mode de réalisation, l'ensemble d'implants peut comprendre des éléments intercalaires disposés de sorte que chaque élément intercalaire relie deux implants entre eux. Dans cette alternative, les implants ne sont pas en contact deux à deux, car chaque élément intercalaire est intercalé entre deux implants. Les éléments intercalaires peuvent être par exemple des capuchons couvrant chacun l'aiguille d'un implant respectif. Ainsi, de tels éléments intercalaires permettent d'isoler et d'envelopper partiellement chaque implant dans l'ensemble d'implants.

Selon un mode de réalisation, chaque implant présente une pointe destinée à pénétrer dans l'épiderme de l'être vivant, et chaque implant présente un logement qui est situé à l'opposé de la pointe et qui est configuré pour loger au moins partiellement la pointe de l'implant suivant.

Ainsi, dans l'ensemble d'implants, les implants sont deux à deux liés entre eux par une liaison de contact surfacique.

Selon une variante, chaque implant présente une pointe en forme de cône, de tétraèdre ou de pyramide. Alternativement à cette variante, chaque implant présente une pointe en forme de harpon, c'est-à-dire avec au moins deux crochets de part et d'autre d'une tige centrale.

Selon une variante, l'ensemble d'implants et le passage sont configurés de sorte que, avant le premier des déplacements successifs, l'implant distal est entièrement situé à l'intérieur du passage. L'implant distal désigne l'implant le plus éloigné du membre de transmission, donc le plus proche de l'orifice de sortie. Ainsi, l'implant distal ne risque pas de toucher un objet extérieur avant d'être posé dans la peau de l'être vivant. On évite ainsi de contaminer l'implant distal avant son implantation.

Dans la présente demande, le terme « distal » désigne un élément qui est relativement éloigné de l'organe de poussée, tandis que le terme « proximal » désigne un élément qui est relativement proche de l'organe de poussée. Par exemple, lorsque le dispositif d'implantation est en service, c'est l'implant distal qui sort en premier par l'orifice de sortie pour pénétrer dans l'épiderme de l'être vivant.

Selon un mode de réalisation, chaque logement est formé par une paroi distale et une ouverture proximale, chaque pointe et chaque logement étant configurés de sorte que l'angle solide défini par une pointe respective est supérieur à un angle solide défini entre l'ouverture proximale et un point quelconque de la paroi distale.

Ainsi, de tels angles solides évitent que l'extrémité de chaque pointe ne touche la paroi distale du logement, ce qui risquerait d'émousser chaque pointe logée dans un logement respectif.

Dans la variante où la translation de l'ensemble d'implants est curviligne, la différence entre :
i) l'angle solide défini par une pointe respective
ii) l'angle solide défini entre l'ouverture proximale et un point quelconque de la paroi distale
est supérieure ou égale à 0,03 sr (stéradians).

Selon une variante, chaque implant est composé d'un matériau sélectionné dans le groupe constitué d'un acier inoxydable de grade médical, d'un alliage de titane de grade médical et d'un matériau biorésorbable. Selon une variante, chaque implant a une longueur comprise entre 1,5 mm et 5 mm, par exemple égale à 3 mm, et une dimension perpendiculaire à la longueur comprise entre 0,5 mm et 3 mm, par exemple égale à 1,3 mm. Ainsi, un tel implant peut former une aiguille d'acupuncture adaptée à l'auriculothérapie.

Selon une variante, l'ensemble d'implants comprend entre 2 et 10 implants, par exemple 5 implants. Ainsi, ce nombre d'implants forme une réserve suffisante pour la plupart des actes que doit accomplir un praticien, en particulier en acupuncture.

Selon un mode de réalisation, l'intervalle entre des extrémités distales de deux implants successifs étant égal à la distance entre des extrémités distales de deux saillies successives.

En d'autres termes, cet intervalle représente le pas selon lequel l'ensemble d'implants avance à chaque déplacement de l'organe de poussée entre la position de repos et la position de poussée.

Ainsi, un tel intervalle permet de stocker les implants dans le dispositif d'implantation de manière relativement compacte selon la ligne d'implants.

Selon une variante, l'intervalle entre des extrémités distales de deux implants successifs étant compris entre 1 mm et 4 mm, par exemple égale à 2,7 mm.

Selon une variante, le passage présente une portion proximale ayant sensiblement une section de dimension constante et une portion distale de section restreinte par rapport à la section de dimension constante. Ainsi, une telle section restreinte permet de retenir l'ensemble d'implants à l'intérieur du passage en l'absence d'effort de poussée.

Selon une variante, la section restreinte est configurée pour être déformée élastiquement lors des franchissements successifs par les implants.

Selon un mode de réalisation, le dispositif d'implantation comprend en outre un organe de rappel agencé pour exercer sur l'organe de poussée l'effort de rappel vers la position de repos.

Ainsi, un tel organe de rappel simplifie la manipulation du dispositif d'implantation, car l'organe de rappel ramène tout seul l'organe de poussée en position de repos.

Alternativement au mode de réalisation précédent, l'organe de poussée comprend une portion de préhension configurée pour permettre au praticien de déplacer l'organe de poussée vers la position de repos. En d'autres termes, le dispositif d'implantation est dépourvu d'organe de rappel. Ainsi, le praticien peut ramener l'organe de poussée en position de repos.

Selon une variante, l'organe de rappel est configuré pour travailler en compression contre l'organe de poussée et contre l'organe de guidage.

Selon une variante, l'organe de rappel est élastiquement déformable.

Alternativement à cette variante, l'organe de rappel peut être configuré pour travailler en traction sur l'organe de poussée et sur l'organe de guidage.

Selon une variante, l'organe de rappel comprend un ressort hélicoïdal. Ainsi, un tel ressort hélicoïdal permet de rappeler l'organe de poussée vers la position de repos de manière répétable et durable, car ce ressort hélicoïdal présente une raideur constante au cours de la durée de service.

Dans la variante où le ressort hélicoïdal est configuré pour travailler en traction, le ressort hélicoïdal peut être dimensionné de sorte que les spires du ressort hélicoïdal sont jointives lorsque le ressort hélicoïdal est au repos. Des spires ainsi jointives permettent un assemblage facile du dispositif d'implantation, notamment car ces spires jointives évitent l'emmêlement de plusieurs ressorts hélicoïdaux dans le conteneur de ressorts hélicoïdaux.

Alternativement à cette variante, l'organe de rappel peut être composé d'un matériau plastique, métallique ou composite. Par exemple, l'organe de rappel peut être composé d'un matériau élastomère ou thermoplastique élastomère. En particulier, l'organe de rappel peut comprendre au moins un matériau sélectionné dans le groupe constitué d'un caoutchouc, d'un latex, d'un composé siliconé et d'un éthylène-propylène-diène monomère (EPDM).

Selon une variante, l'organe de rappel peut être monobloc avec un composant du dispositif d'implantation, par exemple avec l'organe de guidage ou avec l'organe de poussée. Cet organe de rappel monobloc peut être hélicoïdal ou non. Un tel organe de rappel monobloc permet de simplifier le montage du dispositif d'implantation, car il dispense de positionner et d'assembler un organe de rappel indépendant, par exemple un ressort hélicoïdal en acier à ressort.

Selon un mode de réalisation, l'organe de guidage comporte une surface de guidage qui est agencée pour guider l'organe de poussée en translation selon la ligne d'implants.

Ainsi, une telle surface de guidage permet une translation de l'organe de poussée, ce qui lui permet d'exercer l'effort de poussée sur le membre de transmission.

Selon une variante, la surface de guidage est située sur une portion externe de l'organe de guidage.

Selon une variante, la surface de guidage a globalement une forme prismatique ou cylindrique dont l'axe est substantiellement parallèle à la ligne d'implants.

Selon une variante, la surface de guidage présente des cannelures s'étendant substantiellement parallèlement à la ligne d'implants. Ainsi, de telles cannelures permettent d'améliorer le glissement en autorisant une tolérance dimensionnelle.

Selon une variante, le nombre de saillies est supérieur au nombre d'implants, par exemple d'une unité. Ainsi, un tel nombre de saillies garantit au praticien de pouvoir pousser tous les implants contenus dans le dispositif d'implantation.

Selon un mode de réalisation, l'élément d'arrêt unidirectionnel et le membre de transmission sont configurés pour générer un bruit caractéristique, par exemple un cliquetis, lorsque l'élément d'arrêt unidirectionnel passe de la configuration de franchissement à la configuration d'arrêt.

Ainsi, un tel bruit caractéristique indique au praticien que l'élément d'arrêt unidirectionnel a atteint la configuration d'arrêt, si bien que le praticien peut cesser d'exercer l'effort de poussée sur l'organe de poussée.

Selon une variante, la partie d'appui et le membre de transmission sont configurés pour générer un bruit caractéristique, par exemple un cliquetis, lorsque l'élément d'arrêt unidirectionnel passe de la configuration de franchissement à la configuration d'arrêt.

Selon un mode de réalisation, chaque saillie a globalement une forme tronconique ayant une partie étroite et une partie large, la partie étroite étant située plus près de l'orifice de sortie que la partie large.

Ainsi, une telle forme tronconique facilite la fabrication du membre de transmission, l'organe de poussée et l'organe de guidage, car ils présentent des symétries de révolution. De plus, cette symétrie de révolution dispense d'orienter le membre de transmission lors de l'assemblage du dispositif d'implantation.

Selon une variante, le membre de transmission présente deux faces lisses opposées, les saillies étant disposées au moins sur une face s'étendant entre les deux faces lisses opposées, et chaque saillie a globalement une forme de prisme triangulaire ayant une portion étroite et une portion large, la portion étroite étant située plus près de l'orifice de sortie que la portion large.

Dans la présente demande, le terme « lisse » désigne un élément, par exemple une face, qui est dépourvue de saillie. Néanmoins, une face lisse peut être plus ou moins rugueuse. Selon une variante, au moins l'une des deux faces lisses est plane.

Ainsi, un tel organe de transmission peut présenter un encombrement particulièrement faible selon une direction sensiblement perpendiculaire à la ligne d'implants.

Selon une variante, le prisme triangulaire présente, en section transversale, un angle compris entre 4 degrés et 30 degrés, par exemple égal à 8 degrés, l'angle pointant vers l'orifice de sortie.

Selon une variante, les saillies sont disposées sur les deux faces s'étendant entre les deux faces lisses opposées.

Selon un mode de réalisation, l'organe de poussée comprend en outre un conduit configuré pour permettre des coulissements successifs des saillies en translation vers l'orifice de sortie et selon la ligne d'implants.

Ainsi, un tel conduit permet de supporter et de guider le membre de transmission.

Selon un mode de réalisation, l'organe de guidage est translucide ou transparent et dans lequel l'ensemble d'implants et/ou le membre de transmission est(sont) totalement ou partiellement coloré(s).

Ainsi, le praticien peut voir à travers l'organe de guidage le nombre d'implants encore disponibles. Avantageusement, le membre de transmission et/ou l'ensemble d'implants est(sont) coloré(s) en au moins une couleur vive.

Selon une variante, l'organe de guidage, le membre de transmission et/ou l'organe de poussée sont composés de matières plastiques de grade médical. Ainsi, le membre de transmission et/ou l'organe de poussée peuvent être fabriqués par moulage par injection avec des formes complexes et économiques.

Selon un mode de réalisation de la variante précédente, l'organe de rappel est monobloc avec l'organe de poussée ou avec l'organe de guidage, l'organe de rappel étant composé d'un matériau ayant un module d'Young inférieur à 30 GPa, l'organe de rappel présentant des entailles agencées sensiblement transversalement à la ligne d'implants de façon à permettre une déformation élastique de l'organe de rappel.

Ainsi, un tel organe de rappel monobloc permet de réduire les coûts d'assemblage du dispositif d'implantation, en supprimant l'étape de montage d'un organe de rappel indépendant.

Selon une variante, le membre de transmission a un tronçon lisse situé du côté proximal de la saillie la plus proximale.

Ainsi, un tel tronçon lisse, donc dépourvu de saillie, empêche l'organe de poussée d'appuyer contre le membre de transmission lorsqu'il n'y a plus d'implants dans le dispositif d'implantation. En effet, la saillie la plus proximale est la saillie qui permet de pousser le dernier implant hors du dispositif d'implantation. Ensuite, la partie d'appui de l'organe de poussée glisse sur le tronçon lisse sans appuyer contre le membre de transmission, car elle ne peut plus appuyer contre une saillie. Le praticien est ainsi informé que le dispositif d'implantation ne contient plus d'implants.

Alternativement ou complémentairement à cette variante, le membre de transmission a une portion de butée configurée pour buter contre la partie d'appui. Ainsi, une telle portion de butée ne peut pas franchir la partie d'appui, si bien que l'organe de rappel ne peut pas ramener l'organe de poussée en position de repos.

Donc le praticien comprend qu'il n'y a plus d'implants dans le dispositif d'implantation. Le praticien peut alors jeter le dispositif d'implantation usager. En outre, la portion de butée peut produire un bruit caractéristique en butant contre la partie d'appui, ce qui signale au praticien que le dispositif d'implantation est vide.

Selon une variante, la portion de butée peut avoir globalement une forme cylindrique dont le diamètre est supérieur aux dimensions du passage de la partie d'appui en position de franchissement.

Selon une variante, le membre de transmission est composé d'un matériau sélectionné dans le groupe constitué d'un acier inoxydable, d'un alliage de titane et d'un thermoplastique de densité supérieure à 1000 kg/m³ tel qu'un polychlorure de vinyle (PVC), un polycarbonate (PC) ou un acrylonitrile butadiène styrène (ABS).

Selon un mode de réalisation, la partie d'appui comprend au moins deux portions élastiques séparées par au moins une fente.

Ainsi, de telles portions élastiques permettent à la partie d'appui de présenter successivement sa position d'appui, dans laquelle les portions élastiques sont au repos (non déformées), et sa position de franchissement, dans laquelle les portions élastiques sont déformées par une des saillies, ce qui permet à la partie d'appui de franchir la saillie lorsque l'organe de poussée revient en position de repos.

Selon un mode de réalisation, l'élément d'arrêt unidirectionnel comprend au moins deux pattes élastiques séparées par au moins une entaille.

Ainsi, de telles pattes élastiques permettent à l'élément d'arrêt unidirectionnel de présenter successivement i) sa configuration de franchissement, dans laquelle les pattes élastiques sont déformées par une des saillies, et ii) sa configuration d'arrêt, dans laquelle les pattes élastiques sont au repos (non déformées), ce qui permet à l'élément d'arrêt unidirectionnel de buter contre une des saillies et donc d'immobiliser le membre de transmission en sens inverse de la translation vers l'orifice de sortie et selon la ligne d'implants.

Selon une variante, les pattes élastiques sont configurées pour claquer contre avec une saillie respective lorsque l'élément d'arrêt unidirectionnel est en configuration d'arrêt. Ainsi, les pattes élastiques peuvent claquer contre chaque saillie, de façon à générer le bruit caractéristique. En particulier, la raideur des pattes élastiques et la dureté surfacique du membre de transmission sont sélectionnées pour obtenir le bruit caractéristique.

Selon une variante, les portions élastiques sont configurées pour claquer contre avec une saillie respective lorsque l'élément d'arrêt unidirectionnel est en configuration d'arrêt. Ainsi, les portions élastiques peuvent claquer contre chaque saillie, de façon à générer le bruit caractéristique. En particulier, la raideur des portions élastiques et la dureté surfacique du membre de transmission sont sélectionnées pour obtenir le bruit caractéristique.

Selon une variante, l'équipement d'implantation comprend en outre au moins une enveloppe composée en matériau plastique souple entourant l'ensemble d'implants.

Selon une variante, l'équipement d'implantation comprend en outre un suremballage, ladite enveloppe étant disposée dans le suremballage.

Les modes de réalisation et les variantes mentionnés ci-avant peuvent être pris isolément ou selon toute combinaison techniquement possible.

La présente invention sera bien comprise et ses avantages ressortiront aussi à la lumière de la description qui va suivre, donnée uniquement à titre d'exemple non limitatif et faite en référence aux figures annexées, dans lesquelles des signes de références identiques correspondent à des éléments structurellement et/ou fonctionnellement identiques ou similaires. Dans les figures annexées :
- la figure 1 est une vue schématique en perspective d'un dispositif d'implantation conforme à un premier mode de réalisation de l'invention, avant l'implantation d'un premier implant ;
- la figure 2 est une vue schématique en perspective du dispositif d'implantation de la figure 1, pendant l'implantation du premier implant ;
- la figure 3 est une vue du dispositif d'implantation des figures 1 et 2, en perspective éclatée et avec arrachement partiel ;
- la figure 4 est une vue schématique en coupe, suivant le plan IV à la figure 1, du dispositif d'implantation de la figure 1 ;
- la figure 5 est une vue à plus grande échelle du détail V à la figure 4 ;
- la figure 6 est une vue similaire à la figure 4 du dispositif d'implantation de la figure 2, pendant l'implantation du premier implant ;
- la figure 7 est une vue similaire à la figure 6 du dispositif d'implantation de la figure 2, après l'implantation du premier implant ;
- la figure 8 est une vue similaire à la figure 4 du dispositif d'implantation de la figure 2, avant l'implantation du dernier implant ;
- la figure 9 est une vue similaire à la figure 4 du dispositif d'implantation de la figure 2, pendant l'implantation du dernier implant ;
- la figure 10 est une vue similaire à la figure 6 du dispositif d'implantation de la figure 2, après l'implantation du dernier implant ;
- la figure 11 est une vue schématique en perspective d'une partie du dispositif d'implantation de la figure 1, avant la transmission d'un effort de poussée ;
- la figure 12 est une vue similaire à la figure 11, pendant la transmission d'un effort de poussée ;
- la figure 13 est une vue détaillée en perspective de l'organe de guidage appartenant au dispositif d'implantation de la figure 1 ;
- la figure 14 est une vue similaire à la figure 3 d'un dispositif d'implantation conforme à un deuxième mode de réalisation de l'invention ;
- la figure 15 est une vue en perspective assemblée, similaire à la figure 1, du dispositif d'implantation de la figure 14 ;
- la figure 16 est une vue en coupe médiane similaire à la figure 4 du dispositif d'implantation de la figure 14, avant l'implantation du premier implant ;
- la figure 17 est une vue similaire à la figure 16 du dispositif d'implantation de la figure 16, pendant l'implantation du premier implant ;
- la figure 18 est une vue en coupe médiane similaire à la figure 4 d'un dispositif d'implantation conforme à un troisième mode de réalisation de l'invention, avant l'implantation du premier implant ; et
- la figure 19 est une vue similaire à la figure 18 du dispositif d'implantation de la figure 18, pendant l'implantation du premier implant ;
- la figure 20 est une vue en coupe similaire à la figure 4 d'un dispositif d'implantation conforme à un quatrième mode de réalisation de l'invention, pendant l'implantation du premier implant ;
- la figure 21 est une vue en perspective éclatée, similaire à la figure 3, d'un dispositif d'implantation conforme à un cinquième mode de réalisation de l'invention ;
- la figure 22 est une vue en perspective de l'organe de guidage appartenant à un dispositif d'implantation conforme à un sixième mode de réalisation de l'invention ;
- la figure 23 est une vue en perspective du membre de transmission appartenant à un dispositif d'implantation conforme à un septième mode de réalisation de l'invention ; et
- la figure 24 est une vue en coupe d'une partie d'un train d'implant appartenant à un dispositif d'implantation conforme à un huitième mode de réalisation de l'invention.

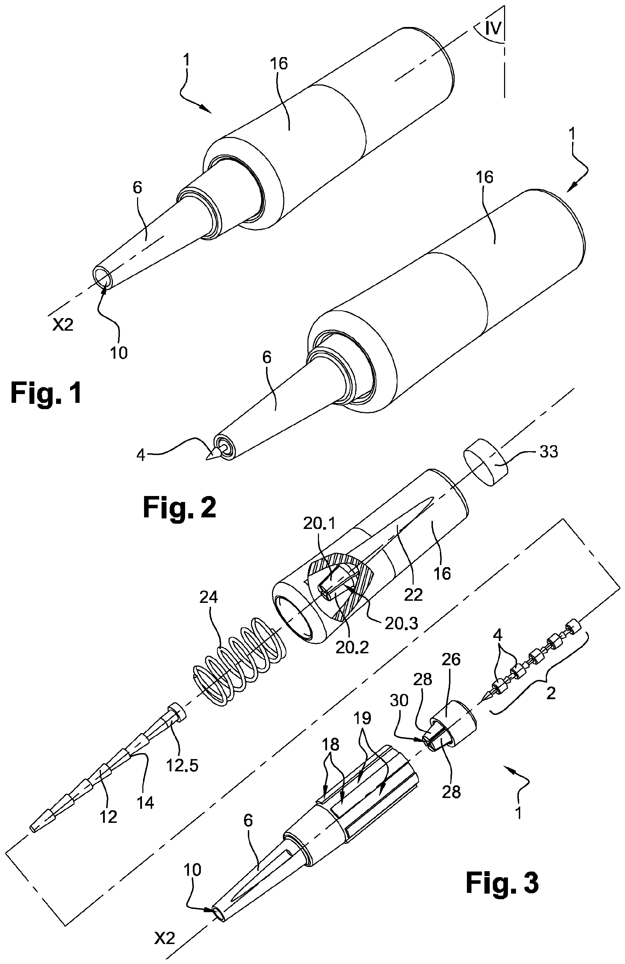

Les figures 1 à 13 illustrent un dispositif d'implantation 1 pour implanter des implants, en l'occurrence des aiguilles d'acupuncture, dans l'épiderme d'un être vivant non représenté.

Le dispositif d'implantation 1 comprend un ensemble d'implants 2. L'ensemble d'implants 2 comprend ici cinq implants 4 disposés successivement selon une ligne d'implants X2. La ligne d'implants X2 est droite.

Les implants 4 sont reliés de sorte que chaque implant 4 peut être séparé de l'ensemble d'implants 2. Les implants 4 sont ici en contact deux à deux, donc contigus, dans l'ensemble d'implants 2.

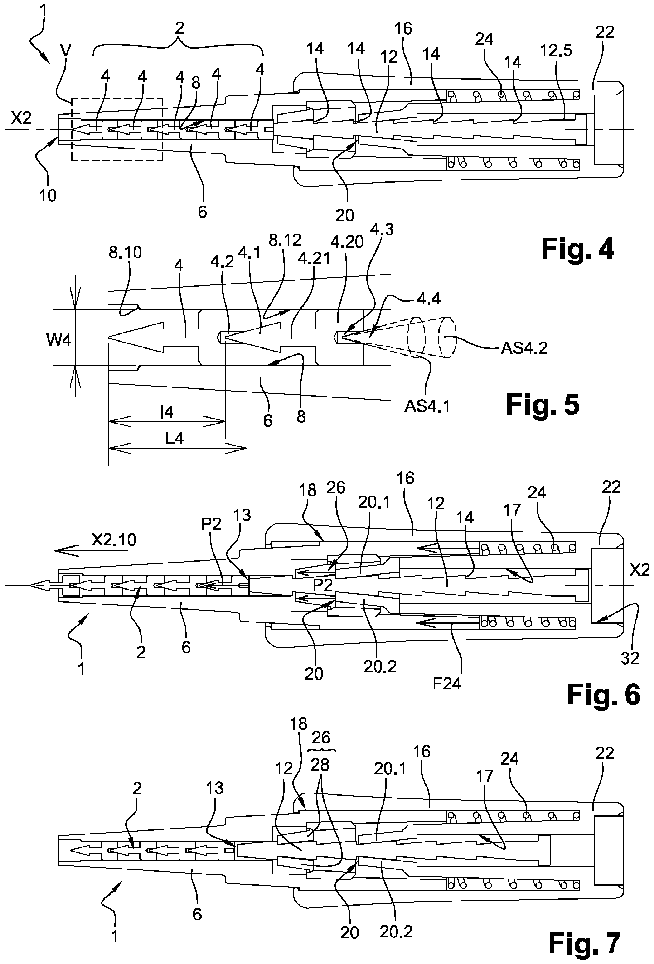

Comme le montre la figure 5, chaque implant 4 a une longueur L4 environ égale à 3 mm et une largeur W4, mesurée perpendiculairement à la longueur L4 environ égale à 1,3 mm. Chaque implant 4 est ici composé d'un acier inoxydable de grade médical.

Comme le montre la figure 5, chaque implant 4 présente une pointe 4.1 destinée à pénétrer dans l'épiderme de l'être vivant. Chaque pointe 4.1 est ici en forme de harpon, c'est-à-dire avec au moins deux crochets de part et d'autre d'une tige centrale.

Chaque implant 4 présente un logement 4.2 qui est situé à l'opposé de la pointe 4.1 et qui est configuré pour loger au moins partiellement la pointe 4.1 de l'implant suivant 4. Chaque logement 4.2 est ménagé dans une embase 4.20 qui est solidarisée à la pointe 4.1 par une tige 4.21. Dans l'ensemble d'implants 2, les implants 4 sont deux à deux liés entre eux par une liaison de contact surfacique.

Chaque logement 4.2 est formé par une paroi distale 4.3 et une ouverture proximale 4.4. Chaque pointe 4.1 et chaque logement 4.2 sont configurés de sorte que l'angle solide AS4.1 défini par une pointe respective est supérieur à un angle solide AS4.2 défini entre l'ouverture proximale 4.4 et un point quelconque de la paroi distale 4.3.

Par exemple, l'angle solide AS4.1 peut être environ égal à 0,214 sr (soit un demi-angle au sommet entre axe et génératrice du cône environ égal à 15 degrés). Par exemple, l'angle solide AS4.2 peut être au maximum égal à 0,185 sr. Ainsi, la différence entre les angles solides AS4.1 et AS4.2 est supérieur ou égale à 0,03 sr (stéradians).

De plus, le dispositif d'implantation 1 comprend un organe de guidage 6, qui est ici composé d'une seule pièce issue du moulage par injection d'un polyéthylène (PE).

L'organe de guidage 6 définit un passage 8. Le passage 8 s'étend selon la ligne d'implants X2 et jusqu'à un orifice de sortie 10. L'orifice de sortie 10 est destiné à être placé contre l'épiderme de l'être vivant. Le passage 8 est configuré pour guider l'ensemble d'implants 2 en translation vers l'orifice de sortie 10 et selon la ligne d'implants X2.

Dans l'exemple des figures 1 à 13, le passage 8 a globalement une forme de cylindre dont l'axe est colinéaire à la ligne d'implants X2 et dont la base a sensiblement les mêmes dimensions transversales (largeur W4) que les implants 4. Le passage 8 est ici directement en contact avec l'ensemble d'implants 2.

Le dispositif d'implantation 1 comprend en outre un membre de transmission 12. Le membre de transmission 12 est agencé pour transmettre un effort de poussée P2 (symbolisé à la figure 6) à l'ensemble d'implants 2 de façon à déplacer l'ensemble d'implants 2 en translation vers l'orifice de sortie 10 et selon la ligne d'implants X2. Le membre de transmission 12 est ici composé d'un polychlorure de vinyle (PVC).

Comme la ligne d'implants X2 est droite, la translation de l'ensemble d'implants 2 est rectiligne. Le membre de transmission 12 a ici une surface distale de poussée 13 qui entre en contact avec l'implant proximal 4 afin de transmettre l'effort de poussée P2 à l'ensemble d'implants 2.

Pour recevoir l'effort de poussée P2, le membre de transmission 12 comporte plusieurs saillies 14. Les saillies 14 sont disposées successivement selon la ligne d'implants X2. En l'occurrence, les saillies 14 sont disposées parallèlement à la ligne d'implants X2. En d'autres termes, le membre de transmission 12 forme une sorte de tige dentelée, dont les dents sont formées par les saillies 14.

Le nombre de saillies 14 est ici supérieur d'une unité au nombre d'implants 4. Dans l'exemple des figures 1 à 13, il y a six saillies 14 et cinq implants 4. Chaque saillie 14 a ici globalement une forme tronconique, avec une partie étroite et une partie large, la partie étroite étant située plus près de l'orifice de sortie 10 que la partie large. En d'autres termes, les surfaces tronconiques des saillies 14 sont orientées vers l'orifice de sortie 10.

De plus, le dispositif d'implantation 1 comprend un organe de poussée 16, qui est composé d'une seule pièce issue du moulage par injection d'un polyéthylène (PE). L'organe de poussée 16 est configuré pour permettre à un praticien de générer l'effort de poussée P2.

L'organe de poussée 16 est déplaçable entre une position de repos (figures 1 et 7) et une position de poussée (figures 2 et 5). 1. À cet effet, l'organe de guidage 6 comporte une surface de guidage 18 qui est agencée pour guider l'organe de poussée 12 en translation selon la ligne d'implants X2.

La surface de guidage 18 permet une translation de l'organe de poussée 16. La surface de guidage 18 est ici située sur une portion externe de l'organe de guidage 6. La surface de guidage 18 a ici globalement une forme cylindrique dont l'axe est parallèle à la ligne d'implants X2.

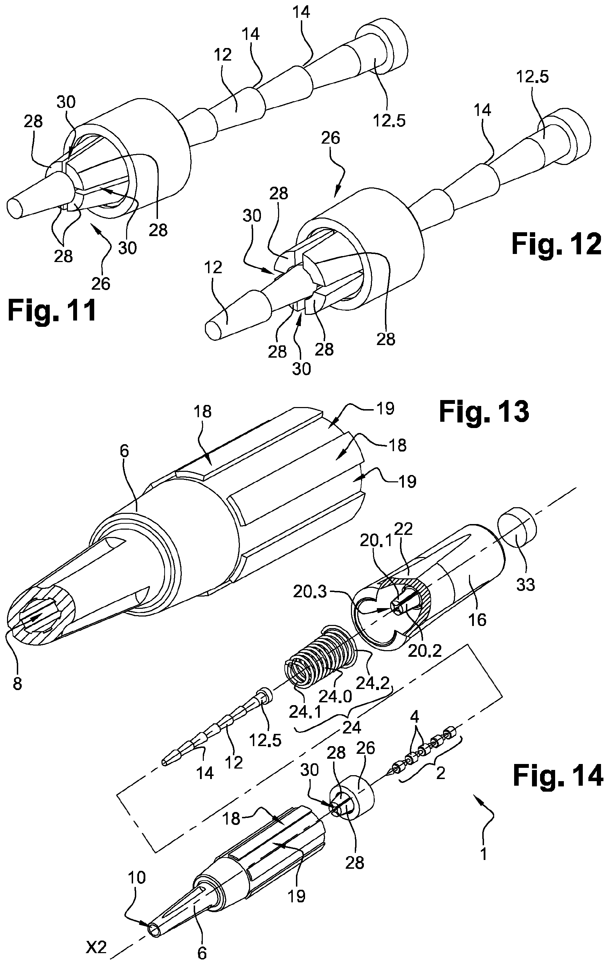

Comme le montre la figure13, la surface de guidage 18 présente des cannelures 19 qui s'étendent substantiellement parallèlement à la ligne d'implants X2. Les cannelures 19 permettent d'améliorer le glissement en autorisant une tolérance dimensionnelle.

L'organe de poussée 16 comporte une partie d'appui 20 qui est configurée pour appuyer successivement contre plusieurs des saillies 14 lors de plusieurs déplacements successifs de l'organe de poussée 16 entre la position de repos (figures 1 et 7) et la position de poussée (figures 2 et 5). La partie d'appui 20 a ici une forme annulaire, car l'organe de poussée 16 a une portion distale cylindrique à base circulaire.

L'ensemble d'implants 2 est configuré de sorte que, avant le premier des déplacements successifs de l'organe de poussée 16, l'implant distal 4 est entièrement situé à l'intérieur du passage 8. L'implant distal 4 désigne l'implant 4 le plus éloigné du membre de transmission 12, donc le plus proche de l'orifice de sortie 10. Ainsi entouré, l'implant distal 4 ne risque pas de toucher un objet extérieur avant d'être posé dans l'épiderme de l'être vivant.

L'intervalle 14 entre des extrémités distales de deux implants successifs 4 est ici régulier et environ égal à 2,7 mm. Cet intervalle 14 entre des extrémités distales de deux implants successifs 4 est égal à la distance entre des extrémités distales de deux saillies successives 14. Cet intervalle représente le pas selon lequel l'ensemble d'implants 4 avance à chaque déplacement de l'organe de poussée 16 entre la position de repos et la position de poussée.

De plus, l'organe de poussée 16 comprend une partie de préhension 22 qui est configurée pour permettre au praticien de manipuler le dispositif d'implantation 1. L'organe de poussée 16 comprend en outre un conduit 17 qui est configuré pour permettre des coulissements successifs des saillies 14 en translation vers l'orifice de sortie 10 et selon la ligne d'implants X2. En service, le conduit 17 supporte et guide le membre de transmission 12.

Le dispositif d'implantation 1 comprend en outre un organe de rappel 24, qui est ici élastiquement déformable. L'organe de rappel 24 est agencé pour exercer sur l'organe de poussée 16 un effort de rappel F24 vers la position de repos (figures 1 et 7).

Dans l'exemple des figures 1 à 13, l'organe de rappel 24 comprend un ressort hélicoïdal qui est configuré pour travailler en compression contre l'organe de poussée 16 et contre l'organe de guidage 6. En service, ce ressort hélicoïdal permet de rappeler l'organe de poussée 16 vers la position de repos.

La partie d'appui 20 comprend ici deux portions élastiques 20.1 et 20.2 qui sont séparées par une fente 20.3. La partie d'appui 20 est configurée pour présenter successivement :
- une position d'appui (figures 4, 6 et 7), dans laquelle la partie d'appui 20 appuie contre une saillie respective 14 lors de chaque déplacement de l'organe de poussée 16 entre la position de repos et la position de poussée (figure 6), et
- une position de franchissement similaire à la position illustrée à la figure 12, dans laquelle la partie d'appui 20 est déformée élastiquement par la saillie respective 14 de sorte que la partie d'appui 20 peut franchir la saillie 14 lorsque l'organe de rappel 24 exerce sur l'organe de poussée 16 l'effort de rappel F24 vers la position de repos.

Les portions élastiques 20.1 et 20.2 permettent à la partie d'appui 20 de présenter successivement :
i) sa position d'appui, dans laquelle les portions élastiques 20.1 et 20.2 sont au repos, donc non déformées, et
ii) sa position de franchissement, dans laquelle les portions élastiques 20.1 et 20.2 sont déformées par une des saillies 14, ce qui permet à la partie d'appui 20 de franchir la saillie 14 lorsque l'organe de poussée 16 revient en position de repos.

La partie d'appui 20 permet, par son appui contre une saillie 14, de transmettre l'effort de poussée P2 de l'organe de poussée 16 au membre de transmission 12, puis, par sa déformation élastique, de laisser l'organe de poussée 16 retourner à sa position de repos (figure 7), avant d'effectuer un nouveau déplacement vers la position de poussée (figure 6).

De plus, le dispositif d'implantation 1 comprend un élément d'arrêt unidirectionnel 26 qui est relié à l'organe de guidage 6 et qui est configuré pour présenter successivement :
- une configuration de franchissement, dans laquelle l'élément d'arrêt unidirectionnel 26 est déformé élastiquement par une des saillies 14 de sorte que la saillie 14 peut franchir l'élément d'arrêt unidirectionnel 26, et
- une configuration d'arrêt (figures 4, 6 et 7), dans laquelle l'élément d'arrêt unidirectionnel 26 bute contre une des saillies 14 de façon à immobiliser le membre de transmission 12 en sens inverse de la translation vers l'orifice de sortie 10 et selon la ligne d'implants X2, c'est-à-dire dans le sens de la flèche X2.10 à la figure 6.

Dans l'exemple des figures 1 à 13, l'élément d'arrêt unidirectionnel 26 est solidaire de l'organe de guidage 6. En l'occurrence, l'élément d'arrêt unidirectionnel 26 et l'organe de guidage 6 sont monoblocs.

Comme le montrent les figures 11 et 12, l'élément d'arrêt unidirectionnel 26 comprend quatre pattes élastiques 28, qui sont séparées par deux entailles 30. Ces pattes élastiques 28 permettent à l'élément d'arrêt unidirectionnel 26 de présenter successivement :
i) sa configuration de franchissement, dans laquelle les pattes élastiques 28 sont déformées par une des saillies 14, et
ii) sa configuration d'arrêt (figures4, 6 et 7), dans laquelle les pattes élastiques 28 sont au repos (non déformées), ce qui permet à l'élément d'arrêt unidirectionnel 26 de buter contre une des saillies 14 et donc d'immobiliser le membre de transmission 12 en sens inverse de la translation vers l'orifice de sortie 10 et selon la ligne d'implants X2.

Le passage 8 présente une portion proximale 8.12 ayant sensiblement une section de dimension constante et une portion distale 8.10 de section restreinte par rapport à la section de dimension constante. En service, la section restreinte permet de retenir l'ensemble d'implants 2 à l'intérieur du passage 8 en l'absence d'effort de poussée P2. La section restreinte est ici configurée pour être déformée élastiquement lors des franchissements successifs par les implants 4.

Par ailleurs, l'élément d'arrêt unidirectionnel 26 et le membre de transmission 12 sont configurés pour générer un bruit caractéristique, ici un cliquetis, lorsque l'élément d'arrêt unidirectionnel 26 passe de la configuration de franchissement à la configuration d'arrêt (figures 4, 6 et 7). En particulier, les pattes élastiques 28 sont configurées pour claquer contre avec une saillie respective 14 lorsque l'élément d'arrêt unidirectionnel 26 est en configuration d'arrêt.

À cet effet, la raideur des pattes élastiques 28 et la dureté surfacique du membre de transmission 12 sont sélectionnées pour obtenir le cliquetis. En service, les pattes élastiques 28 claquent contre chaque saillie 14, ce qui génère le cliquetis.

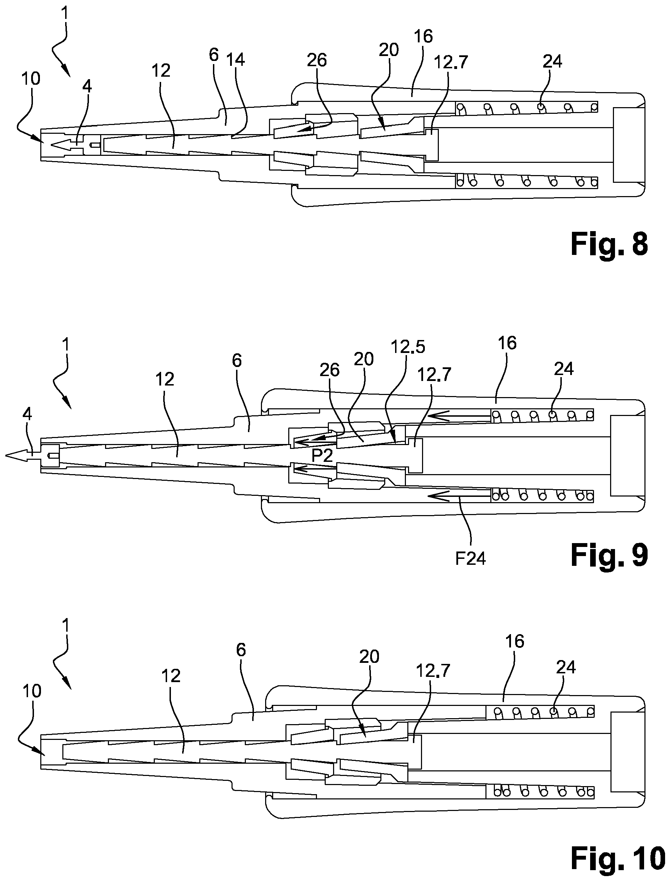

Les figures 8 à 10 illustrent le dispositif d'implantation 1 pendant l'implantation du dernier implant 4 ou implant proximal. Dans cet état, l'ensemble d'implants 2 ne comprend plus qu'un implant 4.

Le membre de transmission 12 présente ici un tronçon lisse 12.5 qui est situé du côté proximal de la saillie 14 la plus proximale. Le tronçon lisse 12.5, dépourvu de saillie 14, empêche l'organe de poussée 16 d'appuyer contre le membre de transmission 12 lorsqu'il n'y a plus d'implants 4 dans le dispositif d'implantation 1.

En effet, la saillie 14 la plus proximale est la saillie qui permet de pousser le dernier implant 4 hors du dispositif d'implantation 1. Ensuite, la partie d'appui 20 glisse sur le tronçon lisse 12.5 sans appuyer contre le membre de transmission 12, car la partie d'appui 20 ne peut plus appuyer contre une saillie 14.

De plus, le membre de transmission 12 a une portion de butée 12.7, qui est configurée pour buter contre la partie d'appui 20. La portion de butée 12.7 ne peut pas franchir la partie d'appui 20, si bien que l'organe de rappel 24 ne peut pas ramener l'organe de poussée en position de repos.

Le praticien comprend qu'il n'y a plus d'implants dans le dispositif d'implantation 1 et il peut alors jeter le dispositif d'implantation usager. Dans l'exemple des figures 8, 9 et 10, la portion de butée 12.7 a globalement une forme cylindrique dont le diamètre est supérieur aux dimensions du passage de la partie d'appui 20 en position de franchissement.

L'organe de guidage 6 peut être translucide et l'ensemble d'implants 2 et/ou le membre de transmission 12 coloré(s). Ainsi, le praticien peut voir à travers l'organe de guidage 6 le nombre d'implants 4 encore disponibles.

Par ailleurs, l'organe de poussée 16 comprend un logement 32, qui peut être utilisé pour loger un aimant 33 visible à la figure 3. Le logement 32 est ici ménagé sur une portion d'extrémité de l'organe de poussée à l'opposé de l'ensemble d'implants 2.

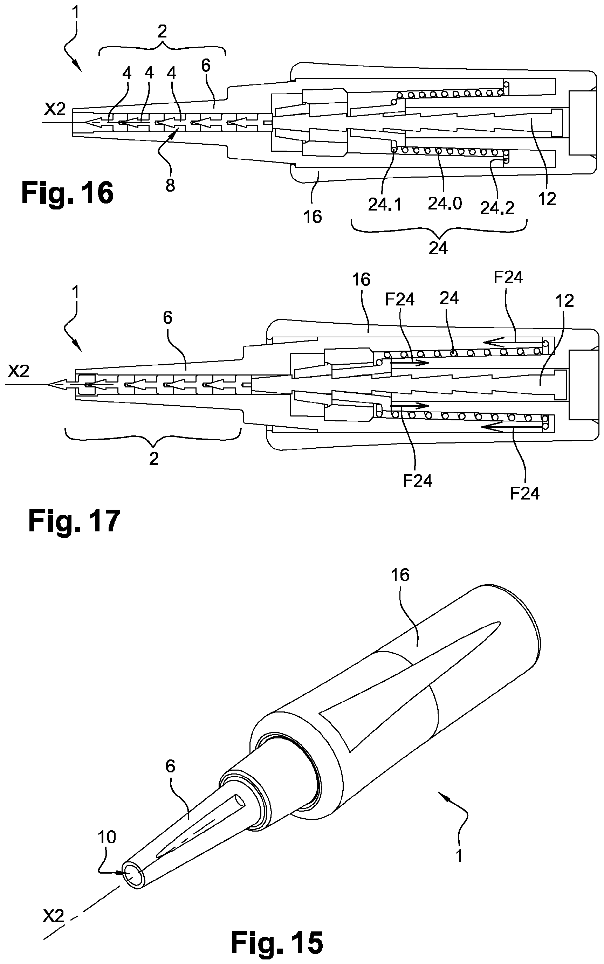

Les figures 14 à 17 illustrent un dispositif d'implantation 1 conforme à un deuxième mode de réalisation. Dans la mesure où le dispositif d'implantation 1 des figures 14 à 17 est similaire au dispositif d'implantation 1 des figures 1 à 13, la description du dispositif d'implantation 1 donnée ci-avant en relation avec les figures 1 à 13 peut être transposée au dispositif d'implantation 1 des figures 14 à 17, à l'exception des différences notables énoncées ci-après.

Le dispositif d'implantation 1 des figures 14 à 17 diffère du dispositif d'implantation 1 des figures 1 à 13, car l'organe de rappel 24 comprend un ressort hélicoïdal qui est configuré pour travailler en traction sur l'organe de poussée 16 et sur l'organe de guidage 6.

L'organe de rappel 24 a ici i) des portions d'extrémités 24.1 et 24.2, qui sont situées respectivement aux deux extrémités de l'organe de rappel, et ii) une portion intermédiaire 24.0 qui s'étend entre les portions d'extrémités 24.1 et 24.2. Chaque portion d'extrémité 24.1 ou 24.2 est formée par une spire terminale. La portion intermédiaire 24.0 comprend plusieurs spires.

La portion d'extrémité 24.1 est agencée en appui contre une surface complémentaire solidaire de l'organe de guidage 6. De même, la portion d'extrémité 24.2 est agencée en appui contre une surface complémentaire solidaire de l'organe de poussée 16 et appartenant ici à la partie d'appui 20. Ainsi, l'organe de rappel 24 est agencé pour travailler en traction.

En service, ce ressort hélicoïdal permet de rappeler l'organe de poussée 16 vers la position de repos. Ce ressort hélicoïdal exerce un effort de rappel F24 sur l'organe de poussée 16 et sur l'organe de guidage 6.

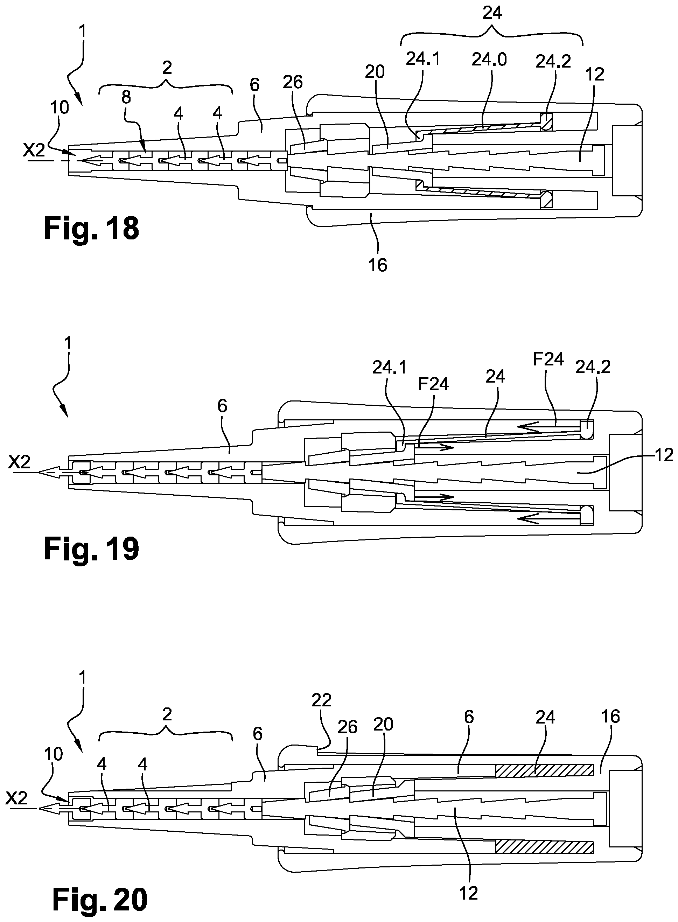

Les figures 18 et 19 illustrent un dispositif d'implantation 1 conforme à un troisième mode de réalisation. Dans la mesure où le dispositif d'implantation 1 des figures 18 et 19 est similaire au dispositif d'implantation 1 des figures 14 à 17, la description du dispositif d'implantation 1 donnée ci-avant en relation avec les figures 14 à 17 peut être transposée au dispositif d'implantation 1 des figures18 et 19, à l'exception des différences notables énoncées ci-après.

Le dispositif d'implantation 1 des figures 18 et 19 diffère du dispositif d'implantation 1 des figures 14 à 17, car l'organe de rappel 24 des figures 18 et 19 est composé d'un matériau élastomère, en l'occurrence du caoutchouc. En service, l'organe de rappel 24 permet de rappeler l'organe de poussée 16 vers la position de repos.

L'organe de rappel 24 a ici i) des portions d'extrémités 24.1 et 24.2, qui sont situées respectivement aux deux extrémités de l'organe de rappel, et ii) une portion intermédiaire 24.0 qui s'étend entre les portions d'extrémités 24.1 et 24.2. Chaque portion d'extrémité 24.1 ou 24.2 a globalement une forme annulaire. La portion intermédiaire 24.0 a globalement une forme tubulaire.

La portion d'extrémité 24.1 est agencée en appui contre une surface complémentaire solidaire de l'organe de guidage 6. De même, la portion d'extrémité 24.2 est agencée en appui contre une surface complémentaire solidaire de l'organe de poussée 16 et appartenant ici à la partie d'appui 20. Ainsi, l'organe de rappel 24 est agencé pour travailler en traction, comme l'organe de rappel 24 du dispositif d'implantation 1 des figures 14 à 17.

Comme le montre la comparaison des figures 18 et 19, l'organe de rappel 24 s'étire lorsque l'organe de poussée est déplacé de façon à pousser un implant 4 hors du passage 8. Cette déformation élastique génère un effort de rappel F24 qui est exercé par la portion annulaire 24.1 sur l'organe de guidage 6 et par la portion annulaire 24.2 sur l'organe de poussée 16.

La figure 20 illustre un dispositif d'implantation 1 conforme à un troisième mode de réalisation. Dans la mesure où le dispositif d'implantation 1 de la figure 20 est similaire au dispositif d'implantation 1 des figures 18 et 19, la description du dispositif d'implantation 1 donnée ci-avant en relation avec les figures 18 et 19 peut être transposée au dispositif d'implantation 1 de la figure 20, à l'exception des différences notables énoncées ci-après.

Le dispositif d'implantation 1 de la figure 20 diffère du dispositif d'implantation 1 des figures 18 et 19, car l'organe de rappel 24 de la figure 20 est agencé pour travailler en compression, comme l'organe de rappel 24 du dispositif d'implantation 1 des figures 1 à 13. L'organe de rappel 24 de la figure 20 est ici formé uniquement d'un tronçon de tube.

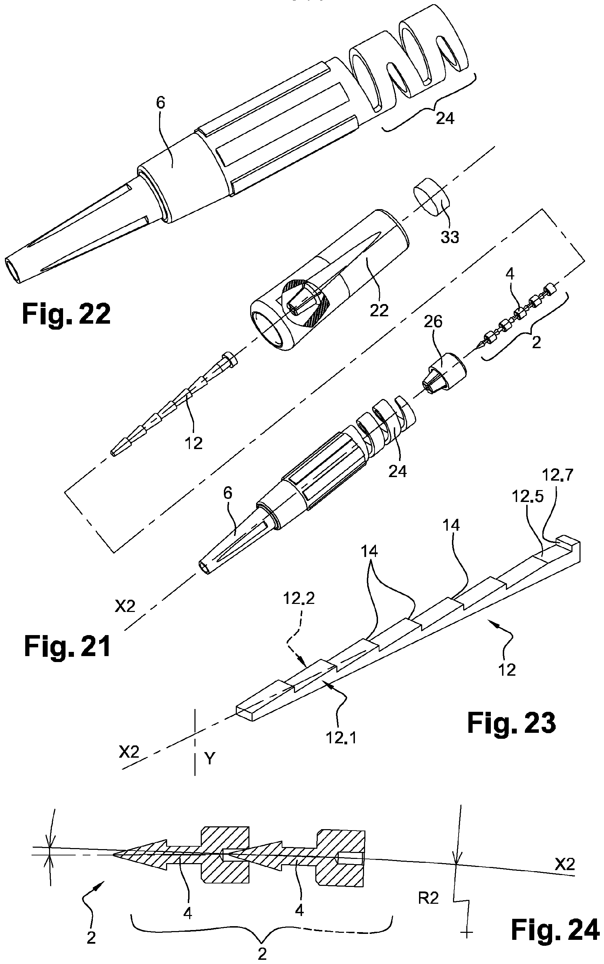

La figure 21 illustre un dispositif d'implantation 1 conforme à un troisième mode de réalisation. Dans la mesure où le dispositif d'implantation 1 de la figure 21 est similaire au dispositif d'implantation 1 des figures 1 à 13, la description du dispositif d'implantation 1 donnée ci-avant en relation avec les figures 1 à 13 peut être transposée au dispositif d'implantation 1 de la figure 21, à l'exception des différences notables énoncées ci-après.

Le dispositif d'implantation 1 de la figure 21 diffère du dispositif d'implantation 1 des figures 1 à 13, car l'organe de rappel 24 de la figure 21 est ici monobloc (donc venu de matière) avec l'organe de guidage 6, ce qui simplifie le montage du dispositif d'implantation 1 de la figure 21.

Comme l'organe de rappel 24 des figures 1 à 13, l'organe de rappel monobloc 24 a une forme hélicoïdale et il est configuré pour travailler en compression entre l'organe de guidage 6 et l'organe de poussée 16. Dans l'exemple de la figure 21, l'organe de rappel 24 et l'organe de guidage 6 sont composés de matière plastique injectable ayant un module d'Young suffisant pour créer la force de rappel, typiquement supérieur à 10 MPa.

La figure 22 illustre une partie d'un dispositif d'implantation conforme à un troisième mode de réalisation. Dans la mesure où le dispositif d'implantation de la figure 22 est similaire au dispositif d'implantation 1 de la figure 21, la description du dispositif d'implantation 1 donnée ci-avant en relation avec la figure 21 peut être transposée au dispositif d'implantation de la figure 22, à l'exception des différences notables énoncées ci-après.

Le dispositif d'implantation de la figure 22 diffère du dispositif d'implantation 1 de la figure 21, car l'organe de rappel 24 de la figure 22 est formé par des spires jointes deux à deux, alors que l'organe de rappel monobloc 24 de la figure 21 a une forme hélicoïdale régulière. La forme particulière de l'organe de rappel 24 de la figure 22 en permet le moulage dans un moule ayant un plan de joint parallèle à la ligne d'implants X2. De plus, la forme particulière de l'organe de rappel 24 de la figure 22 peut accommoder des modules d'Young assez faibles (<10 MPa).

Par ailleurs, l'organe de rappel 24 de la figure 22 est monobloc avec l'organe de guidage 6, comme l'organe de rappel 24 de la figure 21.

La figure 23 illustre une partie d'un dispositif d'implantation conforme à un troisième mode de réalisation. Dans la mesure où le dispositif d'implantation de la figure 23 est similaire au dispositif d'implantation 1 des figures 1 à 13, la description du dispositif d'implantation 1 donnée ci-avant en relation avec les figures 1 à 13 peut être transposée au dispositif d'implantation de la figure 23, à l'exception des différences notables énoncées ci-après.

Le dispositif d'implantation de la figure 23 diffère du dispositif d'implantation 1 des figures 1 à 13, car le membre de transmission 12 présente deux faces lisses opposées 12.1 et 12.2, et car chaque saillie 14 a globalement une forme de prisme triangulaire, alors que le membre de transmission 12 est formé par des saillies 14 de formes tronconiques.

Sur le membre de transmission de la figure 23, les saillies 14 sont disposées sur une face s'étendant entre les deux faces lisses opposées 12.1 et 12.2. Chaque saillie 14 a globalement une forme de prisme triangulaire ayant une portion étroite et une portion large, la portion étroite étant située plus près de l'orifice de sortie 10 que la portion large. Chaque prisme triangulaire présente, en section transversale, un angle qui est environ égal à 8 degrés et qui pointe vers l'orifice de sortie 10.

Ainsi, cet organe de transmission 12 présente un encombrement particulièrement faible selon une direction Y sensiblement perpendiculaire à la ligne d'implants X2.

La figure 24 illustre une partie d'un dispositif d'implantation conforme à un troisième mode de réalisation. Dans la mesure où le dispositif d'implantation de la figure 24 est similaire au dispositif d'implantation 1 des figures 1 à 13, la description du dispositif d'implantation 1 donnée ci-avant en relation avec les figures 1 à 13 peut être transposée au dispositif d'implantation de la figure 24, à l'exception des différences notables énoncées ci-après.

Le dispositif d'implantation de la figure 24 diffère du dispositif d'implantation 1 des figures 1 à 13, car la ligne d'implants X2 est courbe avec une courbure. Lorsque le dispositif d'implantation de la figure 24 est en service, la translation de l'ensemble d'implants 2 est ici curviligne.

Les dimensions et les matériaux des composants du dispositif d'implantation de la figure 24 sont sélectionnés de sorte que l'ensemble d'implants 2 peut glisser dans le passage 8. Le rayon de courbure est ici environ égal à 80 mm. Le membre de poussée non représenté est configuré pour se déformer pendant la translation curviligne de l'ensemble d'implants 2.

Bien entendu, la présente invention n'est pas limitée aux modes de réalisation particuliers décrits dans la présente demande de brevet, ni à des modes de réalisation à la portée de l'homme du métier. D'autres modes de réalisation peuvent être envisagés sans sortir du cadre de l'invention, à partir de tout élément équivalent à un élément indiqué dans la présente demande de brevet.

## Revendications

1. Dispositif d'implantation (1), pour implanter des implants (4), par exemple des aiguilles d'acupuncture du type semi-permanente, dans le corps d'un être vivant, le dispositif d'implantation (1) comprenant au moins :
- un ensemble d'implants (2) comprenant au moins deux implants (4) disposés successivement selon une ligne d'implants (X2), les implants (4) étant reliés de sorte que chaque implant (4) peut être séparé de l'ensemble d'implants (2),
- un organe de guidage (6) définissant un passage (8) qui s'étend selon la ligne d'implants (X2) et jusqu'à un orifice de sortie destiné à être placé contre l'épiderme de l'être vivant, le passage (8) étant configuré pour guider l'ensemble d'implants (2) en translation vers l'orifice de sortie (10) et selon la ligne d'implants (X2),
- un membre de transmission (12) agencé pour transmettre un effort de poussée (P2) à l'ensemble d'implants (2) de façon à déplacer l'ensemble d'implants (2) en translation vers l'orifice de sortie (10) et selon la ligne d'implants (X2), le membre de transmission (12) comportant plusieurs saillies (14) disposées successivement selon la ligne d'implants (X2),
- un organe de poussée (16) configuré pour permettre à un praticien de générer l'effort de poussée (P2), l'organe de poussée (16) étant déplaçable entre une position de repos et une position de poussée, l'organe de poussée (16) comportant une partie d'appui (20) configurée pour appuyer successivement contre plusieurs des saillies (14) lors de plusieurs déplacements successifs de l'organe de poussée (16) entre la position de repos et la position de poussée, la partie d'appui (20) étant configurée pour présenter successivement :
• une position d'appui, dans laquelle la partie d'appui (20) appuie contre une saillie respective (14) lors de chaque déplacement de l'organe de poussée (16) entre la position de repos et la position de poussée, et
• une position de franchissement, dans laquelle la partie d'appui (20) est déformée élastiquement par la saillie respective (14) de sorte que la partie d'appui (20) peut franchir la saillie (14) lorsque l'organe de poussée (16) subit un effort de rappel (F24) vers sa position de repos,
le dispositif d'implantation (1) comprenant en outre un élément d'arrêt unidirectionnel (26) qui est relié à l'organe de guidage (6) et qui est configuré pour présenter successivement :
• une configuration de franchissement, dans laquelle l'élément d'arrêt unidirectionnel (26) est déformé élastiquement par une des saillies (14) de sorte que la saillie (14) peut franchir l'élément d'arrêt unidirectionnel (26), et
• une configuration d'arrêt, dans laquelle l'élément d'arrêt unidirectionnel (26) bute contre une des saillies (14) de façon à immobiliser le membre de transmission (12) en sens inverse de la translation vers l'orifice de sortie (10) et selon la ligne d'implants (X2).

2. Dispositif d'implantation (1) selon la revendication précédente, dans lequel les implants (4) sont en contact deux à deux dans l'ensemble d'implants (2).

3. Dispositif d'implantation (1) selon la revendication précédente, dans lequel chaque implant (4) présente une pointe (4.1) destinée à pénétrer dans l'épiderme de l'être vivant, et dans lequel chaque implant (4) présente un logement (4.2) qui est situé à l'opposé de la pointe (4.1) et qui est configuré pour loger au moins partiellement la pointe (4.1) de l'implant suivant (4).

4. Dispositif d'implantation (1) selon la revendication précédente, dans lequel chaque logement (4.2) est formé par une paroi distale (4.3) et une ouverture proximale (4.4), chaque pointe (4.1) et chaque logement (4.3) étant configurés de sorte que l'angle solide (AS4.1) défini par une pointe (4.1) respective est supérieur à un angle solide (AS4.2) défini entre l'ouverture proximale (4.4) et un point quelconque de la paroi distale (4.3).

5. Dispositif d'implantation (1) selon l'une quelconque des revendications précédentes, dans lequel l'intervalle entre des extrémités distales de deux implants (4) successifs est égal à la distance entre des extrémités distales de deux saillies (14) successives.

6. Dispositif d'implantation (1) selon l'une quelconque des revendications précédentes, comprenant en outre un organe de rappel (24) agencé pour exercer sur l'organe de poussée (16) l'effort de rappel (F24) vers la position de repos.

7. Dispositif d'implantation (1) selon l'une quelconque des revendications précédentes, dans lequel l'organe de guidage (6) comporte une surface de guidage (18) qui est agencée pour guider l'organe de poussée (16) en translation selon la ligne d'implants (X2).

8. Dispositif d'implantation (1) selon l'une quelconque des revendications précédentes, dans lequel l'élément d'arrêt unidirectionnel (26) et le membre de transmission (12) sont configurés pour générer un bruit caractéristique, par exemple un cliquetis, lorsque l'élément d'arrêt unidirectionnel (26) passe de la configuration de franchissement à la configuration d'arrêt.

9. Dispositif d'implantation (1) selon l'une quelconque des revendications précédentes, dans lequel chaque saillie (14) a globalement une forme tronconique ayant une partie étroite et une partie large, la partie étroite étant située plus près de l'orifice de sortie (10) que la partie large.

10. Dispositif d'implantation (1) selon l'une quelconque des revendications précédentes, dans lequel l'organe de poussée (16) comprend en outre un conduit (17) configuré pour permettre des coulissements successifs des saillies (14) en translation vers l'orifice de sortie (10) et selon la ligne d'implants (X2).

11. Dispositif d'implantation (1) selon l'une quelconque des revendications précédentes, dans lequel l'organe de guidage (6) est translucide ou transparent et dans lequel l'ensemble d'implants (2) et/ou le membre de transmission (12) est(sont) totalement ou partiellement coloré(s).

12. Dispositif d'implantation (1) selon la revendication précédente 6, dans lequel l'organe de rappel (24) est monobloc avec l'organe de poussée (16) ou avec l'organe de guidage (6), l'organe de rappel (24) étant composé d'un matériau ayant un module d'Young inférieur à 30 GPa, l'organe de rappel (24) présentant des entailles agencées sensiblement transversalement à la ligne d'implants (X2) de façon à permettre une déformation élastique de l'organe de rappel (24).

13. Dispositif d'implantation (1) selon l'une quelconque des revendications précédentes, dans lequel la partie d'appui (20) comprend au moins deux portions élastiques (20.1, 20.2) séparées par au moins une fente (20.3).

14. Dispositif d'implantation (1) selon l'une quelconque des revendications précédentes, dans lequel l'élément d'arrêt unidirectionnel (26) comprend au moins deux pattes élastiques (28) séparées par au moins une entaille (30).

## Patentansprüche

1. Implantationsvorrichtung (1) zum Implantieren von Implantaten (4), beispielsweise von Akupunkturnadeln, vom Typ teilpermanent, in den Körper eines Lebewesens, wobei die Implantationsvorrichtung (1) mindestens Folgendes umfasst:
- einen Satz an Implantaten (2), der mindestens zwei Implantate (4) umfasst, die nacheinander entlang einer Linie von Implantaten (X2) angeordnet sind, wobei die Implantate (4) derart verbunden sind, dass jedes Implantat (4) von dem Satz an Implantaten (2) getrennt werden kann,
- ein Führungsorgan (6), das einen Durchgang (8) definiert, der sich entlang der Linie von Implantaten (X2) und bis zu einer Ausgangsöffnung erstreckt, dazu bestimmt, an der Oberhaut des Lebewesens platziert zu werden, wobei der Durchgang (8) konfiguriert ist, um den Satz an Implantaten (2) in Translation zu der Ausgangsöffnung (10) und entlang der Linie von Implantaten (X2) zu führen,
- ein Übertragungsglied (12), das angeordnet ist, um eine Schiebekraft (P2) auf den Satz an Implantaten (2) zu übertragen, um den Satz an Implantaten (2) in Translation zu der Ausgangsöffnung (10) und entlang der Linie von Implantaten (X2) zu verschieben, wobei das Übertragungsglied (12) mehrere Überstände (14) beinhaltet, die nacheinander entlang der Linie von Implantaten (X2) angeordnet sind,
- ein Schiebeorgan (16), das konfiguriert ist, um es einem Arzt zu erlauben, die Schiebekraft (P2) zu erzeugen, wobei das Schiebeorgan (16) zwischen einer Ruheposition und einer Schiebeposition verschiebbar ist, wobei das Schiebeorgan (16) ein Abstützteil (20) beinhaltet, das konfiguriert ist, um sich nacheinander an mehreren der Überstände (14) bei mehreren aufeinanderfolgenden Verschiebungen des Schiebeorgans (16) zwischen der Ruheposition und der Schiebeposition abzustützen, wobei das Abstützteil (20) konfiguriert ist, um nacheinander Folgendes aufzuweisen:
• eine Abstützposition, in der sich das Abstützteil (20) an einem entsprechenden Überstand (14) bei jeder Verschiebung des Schiebeorgans (16) zwischen der Ruheposition und der Schiebeposition abstützt, und
• eine Überwindungsposition, in der das Abstützteil (20) durch den entsprechenden Überstand (14) elastisch verformt wird, sodass das Abstützteil (20) den Überstand (14) überwinden kann, wenn das Schiebeorgan (16) eine Rückstellkraft (F24) in seine Ruheposition erfährt,
wobei die Implantationsvorrichtung (1) weiter ein unidirektionales Rastelement (26) umfasst, das mit dem Führungsorgan (6) verbunden ist, und das konfiguriert ist, um nacheinander Folgendes aufzuweisen:
• eine Überwindungskonfiguration, in der das unidirektionale Rastelement (26) durch einen der Überstände (14) elastisch verformt wird, sodass der Überstand (14) das unidirektionale Rastelement (26) überwinden kann, und
• eine Rastkonfiguration, in der das unidirektionale Rastelement (26) an einen der Überstände (14) anschlägt, um das Übertragungsglied (12) in umgekehrter Richtung der Translation zur Ausgangsöffnung (10) und entlang der Linie von Implantaten (X2) zum Stehen zu bringen.

2. Implantationsvorrichtung (1) nach dem vorstehenden Anspruch, wobei die Implantate (4) paarweise im Satz an Implantaten (2) in Kontakt sind.

3. Implantationsvorrichtung (1) nach dem vorstehenden Anspruch, wobei jedes Implantat (4) eine Spitze (4.1) aufweist, die dazu bestimmt ist, in die Oberhaut des Lebewesens einzudringen, und wobei jedes Implantat (4) eine Aufnahme (4.2) aufweist, die sich gegenüber der Spitze (4.1) befindet und die konfiguriert ist, um die Spitze (4.1) des folgenden Implantats (4) mindestens teilweise aufzunehmen.

4. Implantationsvorrichtung (1) nach dem vorstehenden Anspruch, wobei jede Aufnahme (4.2) durch eine distale Wand (4.3) und eine proximale Öffnung (4.4) gebildet wird, wobei jede Spitze (4.1) und jede Aufnahme (4.3) konfiguriert sind, sodass der feste Winkel (AS4.1), der durch eine entsprechend Spitze (4.1) definiert wird, größer als ein fester Winkel (AS4.2) ist, der zwischen der proximalen Öffnung (4.4) und irgendeinem Punkt der distalen Wand (4.3) definiert wird.

5. Implantationsvorrichtung (1) nach einem der vorstehenden Ansprüche, wobei das Intervall zwischen den distalen Enden der beiden aufeinanderfolgenden Implantate (4) gleich einem Abstand zwischen den distalen Enden der beiden aufeinanderfolgenden Überstände (14) ist.

6. Implantationsvorrichtung (1) nach einem der vorstehenden Ansprüche, weiter ein Rückstellorgan (24) umfassend, das angeordnet ist, um auf das Schiebeorgan (16) die Rückstellkraft (F24) zu der Ruheposition auszuüben.

7. Implantationsvorrichtung (1) nach einem der vorstehenden Ansprüche, wobei das Führungsorgan (6) eine Führungsfläche (18) beinhaltet, die angeordnet ist, um das Schiebeorgan (16) in Translation entlang der Linie von Implantaten (X2) zu führen.

8. Implantationsvorrichtung (1) nach einem der vorstehenden Ansprüche, wobei das unidirektionale Rastelement (26) und das Übertragungsglied (12) konfiguriert sind, um ein charakteristisches Geräusch, beispielsweise ein Klicken, zu erzeugen, wenn das unidirektionale Rastelement (26) von der Überwindungskonfiguration in die Rastkonfiguration übergeht.

9. Implantationsvorrichtung (1) nach einem der vorstehenden Ansprüche, wobei jeder Überstand (14) eine im Allgemeinen kegelstumpfförmige Form aufweist, die einen schmalen Teil und einen breiten Teil aufweist, wobei sich der schmale Teil näher an der Ausgangsöffnung (10) befindet, als der breite Teil.

10. Implantationsvorrichtung (1) nach einem der vorstehenden Ansprüche, wobei das Schiebeorgan (16) weiter einen Kanal (17) umfasst, der konfiguriert ist, um aufeinanderfolgende Verschiebungen der Überstände (14) in Translation zur Ausgangsöffnung (10) und entlang der der Linie von Implantaten (X2) zu ermöglichen.

11. Implantationsvorrichtung (1) nach einem der vorstehenden Ansprüche, wobei das Führungsorgan (6) durchscheinend oder durchsichtig ist, und wobei der Satz an Implantaten (2) und/oder das Übertragungsglied (12) vollkommen oder teilweise gefärbt ist (sind).

12. Implantationsvorrichtung (1) nach dem vorstehenden Anspruch 6, wobei das Rückstellorgan (24) in einem Stück mit dem Schiebeorgan (16) oder mit dem Führungsorgan (6) ist, wobei sich das Rückstellorgan (24) aus einem Material zusammensetzt, das ein Young-Modul von weniger als 30 GPa aufweist, wobei das Rückstellorgan (24) Kerben aufweist, die im Wesentlichen derart querlaufend zur Linie von Implantaten (X2) angeordnet sind, um eine elastische Verformung des Rückstellorgans (24) zu ermöglichen.

13. Implantationsvorrichtung (1) nach einem der vorstehenden Ansprüche, wobei das Abstützteil (20) mindestens zwei elastische Abschnitte (20.1, 20.2) umfasst, die durch mindestens einen Spalt (20.3) getrennt sind.

14. Implantationsvorrichtung (1) nach einem der vorstehenden Ansprüche, wobei das unidirektionale Rastelement (26) mindestens zwei elastische Laschen (28) umfasst, die durch mindestens eine Kerbe (30) getrennt sind.

## Claims

1. An implantation device (1), for implanting implants (4) in the body of a living being, for example acupuncture needles of the semi-permanent type, the implantation device (1) comprising at least:
- one set of implants (2) comprising at least two implants (4) successively disposed according to a line of implants (X2), the implants (4) being connected so that each implant (4) can be separated from the set of implants (2),
- one guiding member (6) defining a passage (8) which extends according to the line of implants (X2) and to an outlet orifice intended to be placed against the epidermis of the living being, the passage (8) being configured to guide in translation the set of implants (2) towards the outlet orifice (10) and according to the line of implants (X2),
- one transmission member (12) arranged to transmit a thrust force (P2) to the set of implants (2) so as to displace in translation the set of implants (2) towards the outlet orifice (10) and according to the line of implants (X2), the transmission member (12) including several protrusions (14) successively disposed according to the line of implants (X2),
- one thrust member (16) configured to allow a practitioner to generate the thrust force (P2), the thrust member (16) being displaceable between a rest position and a thrust position, the thrust member (16) including a bearing portion (20) configured to successively bear against several of the protrusions (14) during several successive displacements of the thrust member (16) between the rest position and the thrust position, the bearing portion (20) being configured to successively have:
• a bearing position, in which the bearing portion (20) bears against a respective protrusion (14) during each displacement of the thrust member (16) between the rest position and the thrust position, and
• a crossing position, in which the bearing portion (20) is elastically deformed by the respective protrusion (14) so that the bearing portion (20) can cross the protrusion (14) when the thrust member (16) undergoes a return force (F24) towards its rest position,
the implantation device (1) further comprising a unidirectional stopping element (26) which is connected to the guiding member (6) and which is configured to successively have:
• a crossing configuration, in which the unidirectional stopping element (26) is elastically deformed by one of the protrusions (14) so that the protrusion (14) can cross the unidirectional stopping element (26), and
• a stopping configuration, in which the unidirectional stopping element (26) abuts against one of the protrusions (14) so as to immobilize the transmission member (12) in the opposite direction of the translation towards the outlet orifice (10) and according to the line of implants (X2).

2. The implantation device (1) according to the preceding claim, wherein the implants (4) are in contact in pairs in the set of implants (2).

3. The implantation device (1) according to the preceding claim, wherein each implant (4) has a tip (4.1) intended to penetrate the epidermis of the living being, and wherein each implant (4) has a housing (4.2) which is located opposite to the tip (4.1) and which is configured to at least partially house the tip (4.1) of the next implant (4).

4. The implantation device (1) according to the preceding claim, wherein each housing (4.2) is formed by a distal wall (4.3) and a proximal opening (4.4), each tip (4.1) and each housing (4.3) being configured so that the solid angle (AS4.1) defined by a respective tip (4.1) is greater than a solid angle (AS4.2) defined between the proximal opening (4.4) and any point of the distal wall (4.3).

5. The implantation device (1) according to any one of the preceding claims, wherein the interval between distal ends of two successive implants (4) is equal to the distance between distal ends of two successive protrusions (14).

6. The implantation device (1) according to any one of the preceding claims, further comprising a return member (24) arranged to exert the return force (F24) on the thrust member (16) towards the rest position.

7. The implantation device (1) according to any one of the preceding claims, wherein the guiding member (6) includes a guiding surface (18) which is arranged to guide in translation the thrust member (16) according to the line of implants (X2).

8. The implantation device (1) according to any one of the preceding claims, wherein the unidirectional stopping element (26) and the transmission member (12) are configured to generate a characteristic noise, for example a clicking, when the unidirectional stopping element (26) passes from the crossing configuration to the stopping configuration.

9. The implantation device (1) according to any one of the preceding claims, wherein each protrusion (14) has generally a frusto-conical shape having a narrow portion and a wide portion, the narrow portion being located closer to the outlet orifice (10) than the wide portion.

10. The implantation device (1) according to any one of the preceding claims, wherein the thrust member (16) further comprises a conduit (17) configured to allow successive sliding of the protrusions (14) in translation towards the outlet orifice (10) and according to the line of implants (X2).

11. The implantation device (1) according to any one of the preceding claims, wherein the guiding member (6) is translucent or transparent and wherein the set of implants (2) and/or the transmission member (12) is (are) totally or partially colored.

12. The implantation device (1) according to the preceding claim 6, wherein the return member (24) is integral with the thrust member (16) or with the guiding member (6), the return member (24) being composed of a material having a Young's modulus less than 30 GPa, the return member (24) having notches arranged substantially transversely to the line of implants (X2) so as to allow an elastic deformation of the return member (24).

13. The implantation device (1) according to any one of the preceding claims, wherein the bearing portion (20) comprises at least two elastic portions (20.1, 20.2) separated by at least one slot (20.3).

14. The implantation device (1) according to any one of the preceding claims, wherein the unidirectional stopping element (26) comprises at least two elastic tabs (28) separated by at least one notch (30).
